# EUROPEAN PATENT APPLICATION

(11) **EP 4 721 701 A1**
(43) Date of publication of application: **08.04.2026**
(21) Application number: 24830441.2
(22) Date of filing: 06.06.2024
(51) Int. Cl.: A61F 2/07, D03D 13/00

(54) **COVER MEMBRANE, COVERED STENT AND COVER MEMBRANE MANUFACTURING METHOD**

(30) Priority: 29.06.2023 CN 202310778709
(71) Applicant: Beijing Percutek Therapeutics Inc., Beijing 102609 (CN)
(72) Inventor: LIU, Ying, Beijing 102609 (CN); LUO, Jingyun, Beijing 102609 (CN); DONG, Yonghe, Beijing 102609 (CN); DU, Qingqing, Beijing 102609 (CN)
(74) Representative: Chimini, Francesco
(86) International application number: PCT/CN2024/097679
(87) International publication number: WO 2025/001795

(57) **Abstract**

The present disclosure relates to the technical field of covered stents, and provides a cover membrane, a covered stent and a cover membrane manufacturing method. The cover membrane is provided with a base area and an easy fenestration area; in the base area, any warp yarn is deflected between a surface layer and an inner layer every time the warp yarn is interwoven with j weft yarns, and any weft yarn is deflected between the surface layer and the inner layer every time the weft yarn is interwoven with k warp yarns; and in the easy fenestration area, at least one warp yarn is deflected between the surface layer and the inner layer every time the at least one warp yarn is interwoven with m weft yarns, and at least one weft yarn is deflected between the surface layer and the inner layer every time the at least one weft yarn is interwoven with n warp yarns, wherein one of m and n is greater than one of j and k, and is greater than or equal to the other one of j and k, and the other one of m and n is greater than or equal to j, and is greater than or equal to k. Membrane rupture and fenestration are easy in the easy fenestration area, the cover membrane would not break or fall off, embolism can be prevented, the occurrence of endoleaks can be reduced, and in situ fenestration positions and number and fenestration shapes can be diversified, thereby adapting to variable branch blood vessel physiological structures of patients.

## Description

### CROSS-REFERENCE TO RELATED APPLICATION

The present disclosure claims priority to Chinese patent Application No. 2023107787093, filed with the Chinese Patent Office on June 29th, 2023, entitled "COVER MEMBRANE, COVERED STENT AND COVER MEMBRANE MANUFACTURING METHOD", the entire contents of which are incorporated herein by reference.

### TECHNICAL FIELD

The present disclosure relates to the technical field of stent grafts, and in particular to a cover membrane (graft), a covered stent (stent graft), and a cover membrane manufacturing method (graft manufacturing method).

### BACKGROUND ART

Endovascular repair-because of its minimal invasiveness, rapid recovery, low peri-operative morbidity, and reduced mortality-has become the standard treatment for anatomically suitable thoracic aortic aneurysms, aortic dissections, and abdominal aortic aneurysms. The technique, however, requires adequate proximal and distal landing zones, and many aneurysms or dissections abut critical aortic side branches, rendering conventional endografting contraindicated. Thoracic endovascular aortic repair (TEVAR) with selective revascularization of aortic arch branches is therefore a major clinical and technical challenge: the aneurysm or intimal tear must be excluded while preserving perfusion to the supra-aortic trunks. The aortic arch is anatomically complex; its three-dimensional curvature varies among individuals, and the brachiocephalic, left common carotid, and left subclavian arteries-which supply the brain and upper limbs-arise from it. Aneurysms, penetrating atherosclerotic ulcers, and dissections often involve this territory. With advances in technology and operator experience, several strategies have emerged: hybrid open-endovascular procedures, branched stent grafts, chimney (parallel) grafts, and fenestrated stent grafts. These approaches, however, carry risks of endoleak, technical complexity, increased procedural trauma, and inadvertent branch occlusion, any of which can precipitate serious complications. Similarly, endovascular management of juxtarenal or paravisceral abdominal aortic lesions remains challenging. Fenestrated, chimney, and periscope techniques require customized devices and meticulous deployment, and their long-term durability is uncertain. At present, no simple, reproducible, and safe solution exists for aortic arch or visceral-branch-bearing abdominal aortic lesions, and these anatomical segments continue to limit the applicability of minimally invasive endovascular therapy.

The in-situ fenestration technique consists of deploying a stent graft-graft across the aneurysm segment, then creating a fenestration at the site where the target supra-aortic or visceral branch arises, thereby establishing an endoluminal blood-flow channel between the aorta and the branch vessel to exclude the aneurysm while preserving branch perfusion. All steps are performed entirely in situ. After stent graft deployment in the arch (which temporarily covers the arch branches), retrograde graft puncture is performed from the branch artery with a stiff guidewire tip, radiofrequency probe, laser fibre, or needle; a guidewire rail is established, the graft puncture site is balloon-dilated, and a covered side-arm stent is deployed. The principal challenge is graft puncture: the low-porosity, high-tear-resistance PET fabric resists piercing, whereas reducing graft strength facilitates fenestration but increases porosity and diminishes fatigue and abrasion resistance. During the manoeuvre, the stent graft interrupts cerebral perfusion; triple fenestration occludes all supra-aortic branches, and prolonged graft puncture time raises stroke risk. Post-puncture balloon expansion often leaves short flaps or petal-like edges or propagating tears along the yarn direction, predisposing to type I/III endoleak and compromising graft durability. Retrograde puncture may also injure adjacent structures or embolize graft-material debris. Success and safety are maximized by selecting a perpendicular puncture device, understanding graft geometry pre-operatively, and centering the fenestration within the branch ostium.

However, the primary material currently used for stent grafts in treating lesions involving aortic dissections and aneurysms of branch vessels is polyethylene terephthalate (PET, Dacron^{®}). Such material has high tensile and puncture resistance, making it difficult to perform in-situ fenestration puncture of the graft and necessitating specialized puncture devices, thereby resulting in a complex operation. Further, during retrograde puncture, there is a risk of damaging surrounding tissues or blood vessels and even graft-material fragment detachment leading to distal embolization. When performing in-situ fenestration, the stent graft will interrupt cerebral perfusion (especially during triple fenestration, which occludes all supra-aortic branches). If graft puncture cannot be completed promptly, the incidence of cerebral infarction increases significantly. After fenestration puncture, the opening must be enlarged with a balloon; however, the fenestration edges are prone to short flaps or petal-like edges, or propagating tears along the yarn direction of the woven graft, which not only increases the risk of type I/III endoleak but also compromises graft structural integrity and long-term durability. If a pre-fenestrated stent graft is used, its off-the-shelf configuration has limited adaptability to the variable anatomy of patient branch vessels. Pre-fenestration typically requires yarn ablation at the opening, which weakens the overall fabric strength. During implantation, precise deployment is required; the stent graft may undergo torsion, foreshortening, or incomplete deployment, making accurate coaxial alignment of multiple fenestrations with target vessels difficult to achieve.

### SUMMARY

An objective of the present disclosure is to provide a graft, a stent graft, and a graft manufacturing method to reduce the difficulty of in-situ graft puncture and to avoid the risk of endoleak.

In a first aspect, the present disclosure provides a graft having an abluminal layer and a luminal layer and configured to cover a stent, wherein the graft comprises a basic area and at least one fenestration-friendly area;
the graft is formed by interweaving multiple warp yarns and multiple weft yarns, and the multiple warp yarns and the multiple weft yarns extend reciprocally to the abluminal layer and the luminal layer;
in the basic area, any one of the warp yarns reverses direction once between the abluminal layer and the luminal layer when interwoven with j weft yarns, and any one of the weft yarns reverses direction once between the abluminal layer and the luminal layer when interwoven with k warp yarns; and
in the fenestration-friendly area, at least one of the warp yarns reverses direction once between the abluminal layer and the luminal layer when interwoven with m weft yarns, and at least one of the weft yarns reverses direction once between the abluminal layer and the luminal layer when interwoven with n warp yarns, wherein
one of m and n is greater than one of j and k, and is greater than or equal to the other of j and k; and the other of m and n is greater than or equal to j, and is greater than or equal to k.

In conjunction with the first aspect, the present disclosure provides one possible embodiment of the first aspect, wherein the basic area is configured as a plain weave structure, and
in the fenestration-friendly area, at least one of the m and n is greater than 1.

In conjunction with the first aspect, the present disclosure provides another possible embodiment of the first aspect. On any one of projection planes perpendicular to the multiple warp yarns within the fenestration-friendly area, the number of multiple warp yarns of the basic area is equal to the number of warp yarns of the fenestration-friendly area, and the multiple warp yarns of the basic area extend continuously with the multiple warp yarns of the fenestration-friendly area; and
on any one of projection planes perpendicular to the multiple weft yarns within the fenestration-friendly area, the number of multiple weft yarns of the basic area is equal to the number of weft yarns of the fenestration-friendly area, and the multiple weft yarns of the basic area extend continuously with the multiple weft yarns of the fenestration-friendly area.

In conjunction with the first aspect, the present disclosure provides another possible embodiment of the first aspect. The bending stiffness of the multiple warp yarns in the fenestration-friendly area is less than bending stiffness of the multiple warp yarns in the basic area, and/or, bending stiffness of the multiple weft yarns in the fenestration-friendly area is less than bending stiffness of the multiple weft yarns in the basic area.

In conjunction with the first aspect, the present disclosure provides another possible embodiment of the first aspect. The yarn fineness of the multiple warp yarns in the fenestration-friendly area is less than the yarn fineness of the multiple warp yarns in the basic area.

In conjunction with the first aspect, the present disclosure provides another possible embodiment of the first aspect, where a number of multifilament filaments of the multiple warp yarns in the fenestration-friendly area is less than a number of multifilament filaments of the multiple warp yarns in the basic area.

In conjunction with the first aspect, the present disclosure provides another possible embodiment of the first aspect, where a density of the multiple warp yarns in the fenestration-friendly area is less than a density of the multiple warp yarns in the basic area.

In conjunction with the first aspect, the present disclosure provides another possible embodiment of the first aspect, wherein elastic films are coated on fabric surfaces formed by interweaving the warp yarns and the weft yarns in the fenestration-friendly area.

In a second aspect, the present disclosure provides a stent graft, including: a stent and the graft according to the first aspect; the graft covers the stent;
the stent graft has at least one in-situ fenestration region; and
the fenestration-friendly area covers the in-situ fenestration region.

In conjunction with the second aspect, the present disclosure provides one possible embodiment of the second aspect, wherein the stent is joined to the basic area; or
an axial length of attachment of the stent to the basic area is greater than an axial length of attachment of the stent to the fenestration-friendly area.

In conjunction with one possible embodiment of the second aspect, the present disclosure provides another possible embodiment of the second aspect, where the stent includes circumferential struts extending along the circumference of the stent, the circumferential struts are bent to form peaks and valleys, and
the basic area covers and joins the peaks and the valleys.

In conjunction with another possible embodiment of the second aspect, the present disclosure provides another possible embodiment of the second aspect, where multiple the circumferential struts are arranged at intervals along an axial direction of the stent, and
the in-situ fenestration region is arranged between the two adjacent circumferential struts.

In conjunction with the second aspect, the present disclosure provides another possible embodiment of the second aspect, where multiple fenestration-friendly areas are arranged at intervals along the axial direction of the stent, and multiple fenestration-friendly areas respectively extend along a circumference of the stent.

In conjunction with the second aspect, the present disclosure provides another possible embodiment of the second aspect, wherein the stent graft has three branch regions in total, including: a brachiocephalic artery branch region, a common carotid artery branch region, and a subclavian artery branch region;
at least one of the brachiocephalic artery branch region, the common carotid artery branch region, and the subclavian artery branch region is configured as the pre-fenestration region; and the fenestration-friendly areas cover the remaining branch regions; or
the fenestration-friendly areas cover the brachiocephalic artery branch region, the common carotid artery branch region, and the subclavian artery branch region.

In conjunction with the second aspect, the present disclosure provides another possible embodiment of the second aspect, where the stent graft has four branch regions in total, including a left renal artery branch region, a right renal artery branch region, an inferior mesenteric artery branch region, and a visceral artery branch region, wherein
at least one of the four branch regions is configured as one of the fenestration-friendly areas, and the remaining branch regions are configured as pre-fenestration regions.

In conjunction with the second aspect, the present disclosure provides another possible embodiment of the second aspect, where at least one of the fenestration-friendly areas extends along the circumference or axial direction of the stent and covers at least one of the in-situ fenestration regions.

In a third aspect, the present disclosure provides a graft manufacturing method, including the following steps: forming a graft by interweaving multiple warp yarns and multiple weft yarns, and providing the graft with a basic area and at least one fenestration-friendly area; in the basic area, making any one of warp yarns reverse direction once between the abluminal layer and the luminal layer after being interwoven with j weft yarns, and making any one of weft yarns reverse direction once between the abluminal layer and the luminal layer after being interwoven with k warp yarns; and in the fenestration-friendly area, selecting at least one of the multiple warp yarns to reverse direction once between the abluminal layer and the luminal layer after being interwoven with m weft yarns, and selecting at least one of the weft yarns to reverse direction once between the abluminal layer and the luminal layer after being interwoven with n warp yarns, wherein one of m and n is greater than one of j and k, and is greater than or equal to the other of j and k; and the other of m and n is greater than or equal to j, and is greater than or equal to k .

In conjunction with the graft manufacturing method provided by the three aspects, the method further includes the following steps:
determining positions of multiple branch regions based on an implantation location of the stent graft;
forming the fenestration-friendly area at a position corresponding to at least one of the branch regions; configuring remaining regions as the basic area; and reserving an opening at a position corresponding to remaining branch regions.

The embodiment of the present disclosure includes the following beneficial effects. It adopts the graft with a basic area and at least one fenestration-friendly area. The graft is formed by interweaving multiple warp yarns and multiple weft yarns, and multiple warp yarns and the multiple weft yarns extend reciprocally to the abluminal layer and the luminal layer. In the basic area, any one of the warp yarns reverses direction once between the abluminal layer and the luminal layer when interwoven with j weft yarns, and any one of the weft yarns reverses direction once between the abluminal layer and the luminal layer when interwoven with k warp yarns; and in the fenestration-friendly area, at least one of the warp yarns reverses direction once between the abluminal layer and the luminal layer when interwoven with m weft yarns, and at least one of the weft yarns reverses direction once between the abluminal layer and the luminal layer when interwoven with n warp yarns, wherein one of m and n is greater than one of j and k, and is greater than or equal to the other of j and k; and the other of m and n is greater than or equal to j, and is greater than or equal to k. As a result, the fenestration-friendly area facilitates the graft puncture and fenestration. The success rate of in-situ fenestration is higher, fragmentation and detachment of the graft caused by graft puncture is avoided, and distal embolism is prevented. The yarn structure around the opening of the in-situ fenestration tends to tighten, which is beneficial for reinforcing the graft tissue around the opening and reducing the occurrence of endoleak. Moreover, it is easy to achieve diversified designs for the position, number, and shape of the opening of the in-situ fenestration, which can adapt to the variable physiological structures of the branch vessels of patients.

In order to make the above objectives, features, and advantages of the present disclosure more obvious and easier to understand, the following is a detailed description of preferred embodiments in conjunction with the drawings.

### BRIEF DESCRIPTION OF DRAWINGS

In order to more clearly illustrate the technical solutions in the embodiments of the present disclosure or in the relevant art, the drawings to be used in the description of the embodiments or relevant art will be briefly introduced below. For a person of ordinary skill in the art, other drawings can be obtained based on these drawings without inventive efforts. For a person of ordinary skill in the art, other drawings can be obtained based on these drawings without inventive efforts.
FIG. 1 is a schematic diagram of a partial unfolding of a graft provided by the embodiments of the present disclosure;
FIG. 2 shows a schematic diagram of a fenestration-friendly area of a graft provided by the embodiments of the present disclosure;
FIG. 3 shows a schematic diagram of another fenestration-friendly area of a graft provided by the embodiments of the present disclosure;
FIG. 4 shows a schematic diagram of a stent graft suitable for an aortic arch provided by the embodiments of the present disclosure;
FIG. 5 shows a schematic diagram of a stent graft being implanted in an aortic arch provided by the embodiments of the present disclosure;
FIG. 6 shows a schematic diagram of another stent graft suitable for an aortic arch provided by the embodiments of the present disclosure;
FIG. 7 shows a schematic diagram of another stent graft suitable for an aortic arch provided by the embodiments of the present disclosure;
FIG. 8 shows a schematic diagram of another stent graft suitable for an aortic arch provided by the embodiments of the present disclosure;
FIG. 9 shows a schematic diagram of another stent graft suitable for an aortic arch provided by the embodiments of the present disclosure;
FIG. 10 shows a schematic diagram of another stent graft suitable for an aortic arch provided by the embodiments of the present disclosure;
FIG. 11 shows a schematic diagram of another stent graft suitable for an aortic arch provided by the embodiments of the present disclosure;
FIG. 12 is a schematic diagram of a stent graft suitable for expanding a true lumen and ensuring blood supply to branch vessels provided by the embodiments of the present disclosure;
FIG. 13 is a schematic diagram of another stent graft suitable for expanding a true lumen and ensuring blood supply to branch vessels provided by the embodiments of the present disclosure;
FIG. 14 shows a schematic diagram of a stent graft suitable for an abdominal aorta provided by the embodiments of the present disclosure;
FIG. 15 shows a schematic diagram of another stent graft suitable for an abdominal aorta provided by the embodiments of the present disclosure;
FIG. 16 shows a schematic diagram of another stent graft suitable for an abdominal aorta provided by the embodiments of the present disclosure;
FIG. 17 shows a schematic diagram of a stent graft being implanted in an abdominal aorta provided by the embodiments of the present disclosure; and
FIG. 18 shows a partial schematic diagram of a stent graft provided by the embodiments of the present disclosure.

Reference numbers: 100-graft; 101-opening; 110-warp yarn; 120-weft yarn; 200-stent; 300-pre-fenestration region; 400-in-situ fenestration region; 500-radiopaque device; 501-first radiopaque part; 502-second radiopaque part; 503-third radiopaque part; 504-fourth radiopaque part; 505-fifth radiopaque part; 506-sixth radiopaque part; 507-seventh radiopaque part; 010-basic area; 020-fenestration-friendly area; 201-peak; 202-valley.

### DETAILED DESCRIPTION OF EMBODIMENTS

The technical solutions in the embodiments of the present disclosure will be clearly and completely described below in conjunction with the drawings in the embodiments of the present disclosure. It is obvious that the embodiments described are partial embodiments of the present disclosure, and not all of the embodiments. Based on the embodiments in the present disclosure, all other embodiments obtained by a person of ordinary skill in the art without inventive efforts shall fall within the scope of protection of the present disclosure.

In the description of the present disclosure, it is to be noted that orientation or positional relationships indicated by terms, such as "center", "up", "down", "left", "right", "vertical", "horizontal", "inside", "outside", are the orientation or positional relationships based on the drawings. The terms are only to facilitate the description of the present disclosure and simplify the description, and are not to indicate or imply that the device or element referred to must have a particular orientation, or be constructed and operated with a particular orientation, and are not to be understood as limitations of the present disclosure. Additionally, the terms "first", "second", and "third", etc., are configured only for descriptions, and are not to be understood as indicating or implying a relative importance. The physical quantities in the formula, if not separately marked, should be understood as the base quantities of the base units in the International System of Units, or the derived quantities derived from the base quantities through mathematical operations such as multiplication, division, differentiation or integration.

In the description of the present disclosure, it should be noted that unless other expressly specifications and limitations, the terms "mount", "connect", and "link" are to be understood in a broad sense, e.g. it can be a fixed connection, a detachable connection, or an integral connection; it can be a mechanical connection or an electrical connection; and it can be a direct connection, an indirect connection through an intermediate medium, or a communication inside two components. For a person of ordinary skill in the art, the specific meaning of the above terms in the present disclosure can be understood according to specific situations.

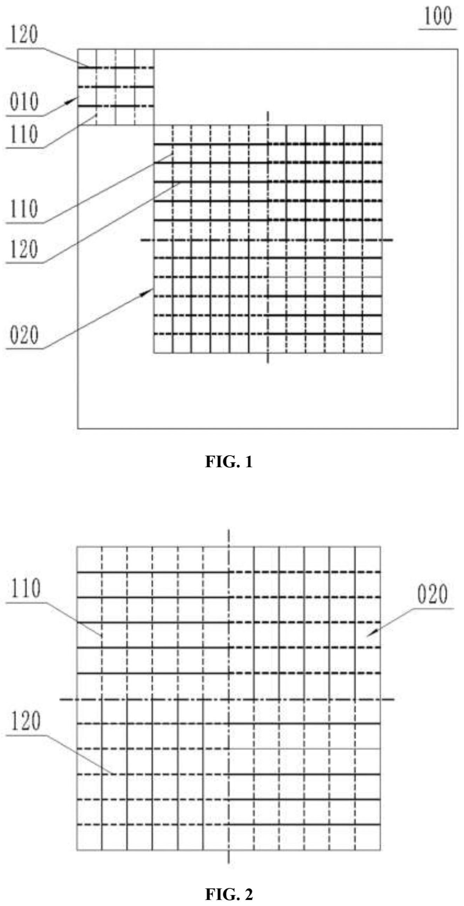

As shown in FIGS. 1, 2, 3, and 4, the embodiments of the present disclosure provide a graft having an abluminal layer and a luminal layer, and configured to cover a stent 200. The graft 100 is provided with a basic area 010 and at least one fenestration-friendly area 020. The graft 100 is formed by multiple warp yarns 110 and multiple weft yarns 120, the multiple warp yarns 110 and multiple weft yarns 120 are interwoven, and the multiple warp yarns 110 and the multiple weft yarns 120 respectively extend back and forth to the abluminal layer and the luminal layer.

In the basic area 010, any one of the warp yarns 110 reverses direction once between the abluminal layer and the luminal layer when interwoven with j weft yarns 120, and any one of the weft yarns 120 reverses direction once between the abluminal layer and the luminal layer when interwoven with k warp yarns 110.

In the fenestration-friendly area 020, at least one of the multiple warp yarns 110 reverses direction once between the abluminal layer and the luminal layer when interwoven with m weft yarns 120, and at least one of the weft yarns 120 reverses direction once between the abluminal layer and the luminal layer when interwoven with n warp yarns 110.

One of m and n is greater than one of j and k, and is greater than or equal to the other of j and k, and the other of m and n is greater than or equal to j, and is greater than or equal to k. It can be understood that the multiple warp yarns 110 and multiple weft yarns 120 in the basic area 010 interweave and reciprocate repeatedly between the abluminal layer and the luminal layer, thereby forming a more dense and compact weaving structure. In the fenestration-friendly area 020, the frequency of warp yarns 110 and weft yarns 120 interweaving and reciprocating between the abluminal layer and luminal layer per unit region is slightly lower compared to the basic area 010. In other words, although the number of warp yarns 110 and weft yarns 120 extending from the basic area 010 to the fenestration-friendly area 020 can remain unchanged, the interweaving of warp yarns 110 and weft yarns 120 in the fenestration-friendly area 020 is sparser and looser. The interweaving density in the fenestration-friendly area 020 is lower than that in the basic area 010. Therefore, when performing the graft puncture in the fenestration-friendly area 020, the weft yarn 120 can deviate along the extension direction of the warp yarn 110, and the warp yarn 110 can also deviate along the extension direction of the weft yarn 120. The deviation and deformation are more likely to occur, and thus, the fenestration operation can be achieved without damaging the warp yarn 110 and weft yarn 120 and without reducing the overall structural strength of the graft 100. Compared with the fenestration-friendly area 020, the basic area 010 only differs in the weaving form. There is no need to change the yarn material, and it is easy to obtain the fenestration-friendly area 020 of the desired shape as needed. Therefore, the fenestration-friendly area 020 can be configured to be rectangular, circular, triangular, rhombic, or elliptical, etc. The diversity in fenestration shapes makes it more adaptable for manufacturing stent grafts of various types of stents. Furthermore, it is easy to configure corresponding quantities, positions, and sizes of the fenestration-friendly areas 020 according to needs, which can accommodate the variable physiological structure of branch vessels of patients. In addition, when performing the in-situ fenestration on in the fenestration-friendly area 020, the graft will not be broken and detached, which can prevent the distal embolism. The yarn structures around the opening of the in-situ fenestration tend to tighten, and the yarn density around the opening is higher, which is beneficial for reinforcing the graft tissue around the opening and reducing the occurrence of endoleak. Moreover, the opening after the graft puncture is reversible. When it is found that the position of the opening is not ideal after the graft puncture, the graft puncture and expansion can be re-performed near the original window, which is beneficial for improving the success rate of the surgery.

In the embodiments of the present disclosure, the basic area 010 is configured as a plain weave structure. In other words, in the basic area 010, any one of the warp yarns 110 reverses direction once between the abluminal layer and the luminal layer when interwoven with one of multiple the weft yarns 120, and any one of the weft yarns 120 reverses direction once between the abluminal layer and the luminal layer when interwoven with one of the multiple warp yarns 110. In the basic area 010, each time one of the multiple warp yarns 110 and one of the multiple weft yarns 120 cross, they will undergo a bend and reverse direction between the abluminal layer and luminal layer. A warp yarn 110 located on the abluminal layer of one weft yarn 120 extends to the luminal layer of another adjacent weft yarn 120, and a weft yarn 120 located on the abluminal layer of one warp yarn 110 extends to the luminal layer of another adjacent warp yarn 110. Thereby, the interweaving of the warp yarns 110 and weft yarns 120 in the basic area 010 becomes denser, forming compact and fine interwoven units, which can better cover the stent 200.

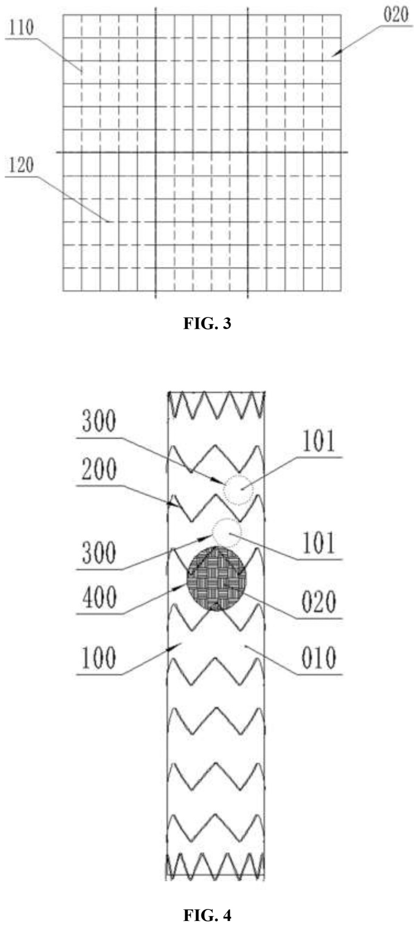

In the fenestration-friendly area 020, at least one of m and n is greater than 1. In other words, in the fenestration-friendly area 020, at least one of the multiple warp yarns 110 reverses direction once between the abluminal layer and the luminal layer when interwoven with two or more weft yarns 120, and/or, at least one of the weft yarns 120 reverses direction once between the abluminal layer and the luminal layer when interwoven with two or more warp yarns 110. Referring to FIGS. 1, 2, and 3, the weft yarns 120 extend in the transverse direction and the warp yarns 110 extend in the longitudinal direction. The warp yarns 110 and the weft yarns 120 are shown as solid lines when extending to the abluminal layer, and are shown as dashed lines when extending to the luminal layer. In the fenestration-friendly area 020 as shown in FIG. 2, five warp yarns 110 and five weft yarns 120 form one group, and two groups of warp yarns 110 and two groups of weft yarns 120 are interwoven to form the smallest interwoven unit in the fenestration-friendly area 020. In the fenestration-friendly area 020 as shown in FIG. 3, four warp yarns 110 and five weft yarns 120 form one group, and two groups of warp yarns 110 and two groups of weft yarns 120 are interwoven to form the smallest interwoven unit in the fenestration-friendly area 020. The number of warp yarns 110 in each group and the number of warp yarns 120 in each group can be increased or decreased according to the requirements for the ease of graft puncture and prevention of endoleak. Increasing the number of warp yarns 110 and warp yarns 120 in each group will make the graft puncture easier, and properly reducing the number of warp yarns 110 and warp yarns 120 in each group appropriately will make the tightening force of yarn structures around the opening greater, thus better preventing endoleak. In a preferred embodiment, three warp yarns 110 form one group, and a single weft yarn 120 forms one group. Two groups of warp yarns 110 and two groups of weft yarns 120 are interwoven to form the smallest interwoven unit in the fenestration-friendly area 020. The three warp yarns 110 are interwoven above one group of the weft yarns 120 and extend below the other group of weft yarns 120. In the extension direction of the weft yarns 120, the three weft yarns 120 are interwoven above one group of the warp yarns 110 and extend below the other group of warp yarns 110. At this time, the fenestration-friendly area 020 has advantages of easy graft puncture and fenestration, a higher success rate of in-situ fenestration, and a lower risk of endoleak.

In an optional embodiment, an implementation of reducing the number of warp yarns 110 and weft yarns 120 within the fenestration-friendly area 020 can be considered, but it needs to consider the technical problem of an increased risk of endoleak due to too few warp yarns 110 and weft yarns 120.

In the embodiment, on any one of projection planes perpendicular to the warp yarns 110 within the fenestration-friendly area 020, the number of warp yarns 110 from the basic area 010 is equal to the number of warp yarns 110 from the fenestration-friendly area 020, and the warp yarns 110 of the basic area 010 extend continuously with the warp yarns 110 of the fenestration-friendly area 020; and on any one of projection planes perpendicular to the weft yarns 120 within the fenestration-friendly area 020, the number of weft yarns 120 from the basic area 010 is equal to the number of weft yarns 120 from the fenestration-friendly area 020, and the weft yarns 120 of the basic area 010 extend continuously with the weft yarns 120 of the fenestration-friendly area 020. In other words, the density of the warp yarns 110 and the weft yarns 120 in the basic area 010 and the fenestration-friendly area 020 are the same, and the same number of warp yarns 110 and weft yarns 120 are interwoven to form the basic area 010 and the fenestration-friendly area 020. The only difference between the basic area 010 and the fenestration-friendly area 020 lies in the structure of the smallest interwoven unit. In the smallest interwoven unit of the basic area 010, any one of the warp yarns 110 reverses direction once between the abluminal layer and the luminal layer when interwoven with one of the weft yarns 120, and any one of the weft yarns 120 reverses direction once between the abluminal layer and the luminal layer when interwoven with one of the warp yarns 110. In the fenestration-friendly area 020, at least one of the warp yarns 110 reverses direction once between the abluminal layer and the luminal layer when interwoven with two or more weft yarns 120, and at least one of the weft yarns 120 reverses direction once between the abluminal layer and the luminal layer when interwoven with two or more warp yarns 110. As a result, without changing the number and material of the warp yarns 110 and weft yarns 120, it is easier to perform the graft puncture operation in the fenestration-friendly area 020 compared to the basic area 010.

Optionally, the bending stiffness of the warp yarns 110 in the fenestration-friendly area 020 is less than the bending stiffness of the warp yarns 110 in the basic area 010, and/or, the bending stiffness of the weft yarns 120 in the fenestration-friendly area 020 is less than the bending stiffness of the weft yarns 120 in the basic area 010. In an optional embodiment, the bending stiffness of the warp yarns 110 and the weft yarns 120 in the fenestration-friendly area 020 is less than the bending stiffness of the warp yarns 110 and the weft yarn 120 in the basic area 010. Optionally, the bending stiffness of the weft yarns 120 in the fenestration-friendly area 020 is less than the bending stiffness of the weft yarns 120 in the basic area 010. Optionally, the bending stiffness of the warp yarns 110 in the fenestration-friendly area 020 is less than the bending stiffness of the warp yarns 110 in the basic area 010. Under the condition that at least one of the warp yarns 110 and the weft yarn 120 within the fenestration-friendly area 020 is prone to deformation, the fenestration of the fenestration-friendly area 020 can be performed more easily.

Optionally, the warp yarns 110 in the fenestration-friendly area 020 are made more prone to bending and deformation compared to the warp yarns 110 in the basic area 010. In an optional implementation, the yarn fineness of the warp yarns 110 in the fenestration-friendly area 020 is less than the yarn fineness of the warp yarns 110 in the basic area 010. In another optional implementation, the yarn count of the warp yarns 110 in the fenestration-friendly area 020 is less than the yarn count of the warp yarns 110 in the basic area 010. In another optional implementation, the density of the warp yarns 110 in the fenestration-friendly area 020 is less than the density of the warp yarns 110 in the basic area 010. All of the above three implementations can make the warp yarns 110 in the fenestration-friendly area 020 more prone to bending and deformation compared to the warp yarns 110 in the basic area 010, so as to easily and successfully perform the graft puncture in the in-situ fenestration region 400.

Optionally, an elastic film is coated on a fabric surface which is formed by interweaving the warp yarns 110 and the weft yarns 120 in the fenestration-friendly area 020. The elastic film can be made of TPU membrane, EPTFE membrane, or silicone membrane, etc. By using the elastic film, the fabric structure within the fenestration-friendly area 020 becomes more compact, thereby enhancing the contraction force of the opening and reducing the risk of endoleak.

As shown in FIG. 4, FIG. 6, FIG. 7, FIG. 8, FIG. 9, FIG. 10, FIG. 11, FIG. 12, FIG. 13, FIG. 14, FIG. 15, and FIG. 16, the embodiments of the present disclosure provide a stent graft, including: a stent 200 and the graft recorded in the above embodiments. The graft 100 covers the stent 200; the stent graft has at least one in-situ fenestration region 400; and the fenestration-friendly area 020 covers the in-situ fenestration region 400.

Optionally, the stent graft can be provided with two pre-fenestration regions 300 and one in-situ fenestration region 400; or the stent graft is provided with one pre-fenestration region 300 and two in-situ fenestration regions 400, and an opening 101 of the graft 100 is formed at the position of the pre-fenestration region 300. It can also cover multiple branch regions of the stent graft through an in-situ fenestration region 400 without providing the pre-fenestration region 300.

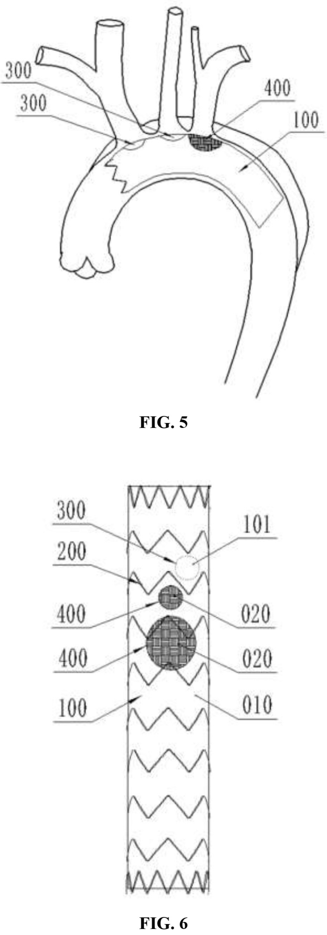

Referring to FIG. 4, the pre-fenestration region 300 is disposed at the branch position of the brachiocephalic artery and the left common carotid artery. The method of pre-fenestration allows for better localization and ensures the blood supply to the branch vessels. The left subclavian artery with significant differences in the lesion positions is configured as the original fenestration area 400. The yarn weaving method of the fenestration-friendly area 020 is adopted to facilitate the graft puncture and forming an in-situ fenestration corresponding to the lesion. Moreover, the size, shape, and position of the opening can be adjusted during the graft puncture process according to the lesion condition. Referring to FIG. 5, the stent graft is implanted into the aortic arch. Most of the branches of the brachiocephalic artery and the left common carotid artery have small differences in location, and the pre-fenestration or branch stent method can accommodate the lesion locations in the majority of patients. The locations of the left subclavian arteries in different patients vary significantly. Therefore, the left subclavian arteries are opened by using the in-situ fenestration method, so as to ensure that the opening can cover the lesion location.

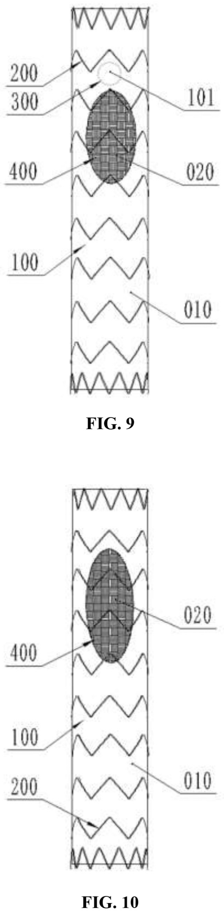

As shown in FIG. 4, FIG. 6, FIG. 7, FIG. 8, and FIG. 9, in optional embodiments, the stent graft has three branch regions in total, including: a brachiocephalic artery branch region, a common carotid artery branch region, and a subclavian artery branch region, wherein at least one of the brachiocephalic artery branch region, the common carotid artery branch region, and the subclavian artery branch region is configured as the pre-fenestration region 300; and the fenestration-friendly areas 020 cover the remaining branch regions. As shown in FIG. 10, the fenestration-friendly area 020 covers the brachiocephalic artery branch region, the common carotid artery branch region, and the subclavian artery branch region, so that each branch of the stent graft can be covered by the fenestration-friendly area 020, thereby facilitating the in-situ fenestration operations to be performed at each branch location respectively.

Since most of the branches of the brachiocephalic artery and the left common carotid artery have smaller differences in location, one or two of the branches can be selected for positioning. The method of pre-fenestration ensures the blood supply to the branch vessels, and the direction of one or two of the above branches is set according to the physiological tissues of the affected region. For other branch regions, the yarn weaving method of the fenestration-friendly area 020 is adopted, which is convenient for the in-situ fenestration, has strong selectivity, and is simple to operate.

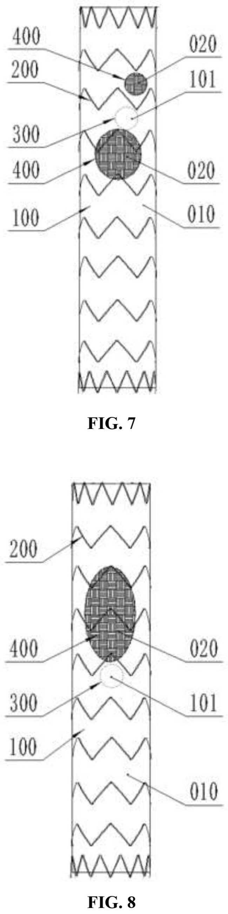

Referring to FIG. 8, in another optional embodiment, the left subclavian branch is configured as a pre-fenestration region 300, and the remaining two branch regions are covered by a large fenestration-friendly area 020.

Referring to FIG. 9, in an optional embodiment, the location of the brachiocephalic artery is selected as a pre-fenestration region 300, and then two branch regions of the left common carotid artery and the left subclavian are covered by a large fenestration-friendly area 020.

Referring to FIG. 10, a large fenestration-friendly area 020 can also be used to cover the brachiocephalic artery branch region, the common carotid artery branch region, and the subclavian artery branch region, thereby allowing each branch to serve as an in-situ fenestration region 400.

As shown in FIG. 9, FIG. 10, FIG. 12, and FIG. 13, at least one of the fenestration-friendly areas 020 extends along the circumference or axial direction of the stent 200 and covers at least one of the in-situ fenestration regions 400. Therefore, a single larger-sized fenestration-friendly area 020 can be used to cover multiple adjacent in-situ fenestration regions 400. The fenestration-friendly area 020 can extend along the axial direction of the stent 200 to form an elliptical, rectangular, or other irregular shape. The fenestration-friendly area 020 can further extend around the entire circumference or half-circle of the stent 200. The in-situ fenestration region of 400 is more easily aligned with the branch vessels and has a stronger adaptability to different physiological structures.

As shown in FIGS. 4, 6, 7, 8, 9, 10, 11, 12, 13, 14, 15, and 16, preferably, the stent 200 is joined to the basic area 010; or an axial length of attachment of the stent 200 to the basic area 010 is greater than an axial length of attachment of the stent 200 to the fenestration-friendly area 020. Since the basic area 010 is structurally more stable than the fenestration-friendly area 020, the stent 200 is joined to the basic area 010 by bonding or suturing, which can better ensure the stable connection between the graft 100 and the stent 200.

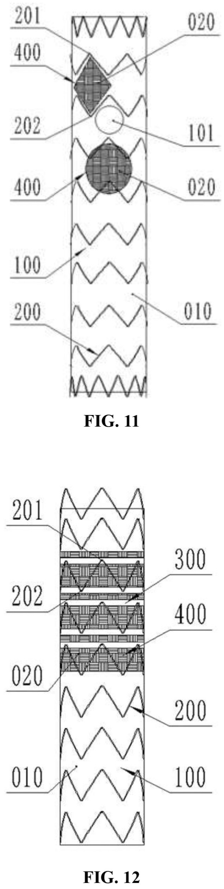

As shown in FIG. 11, FIG. 12, and FIG. 13, the stent 200 includes circumferential struts extending along its circumference, wherein the circumferential struts are bent to form peaks 201 and valleys 202, and the basic area 010 covers and joins the peaks 201 and the valleys 202. The peaks 201 and valleys 202 of the circumferential struts are all covered by the basic area 010. Therefore, the graft puncture operation in the fenestration-friendly area 020 will not cause the peaks 201 and valleys 202 to be exposed. This not only prevents the peaks 201 and valleys 202 from contacting the blood vessel wall with a smaller contact surface area, thereby reducing the stimulation to the blood vessel wall, but also enhances mechanical properties of the stent graft, such as its fatigue resistance.

Referring to FIG. 11, when the stent 200 is in a state where its axis is vertical, in two adjacent circumferential struts, the peak 201 of the upper support rib is opposite to the valley 202 of the lower support rib, and a rhombic in-situ fenestration region 400 can be arranged between the peak 201 and the valley 202, so as to make full use of the membrane covering region between the two circumferential struts. It can obtain a larger area of the in-situ fenestration region 400, and will not result in the exposure of the circumferential struts when performing the graft puncture.

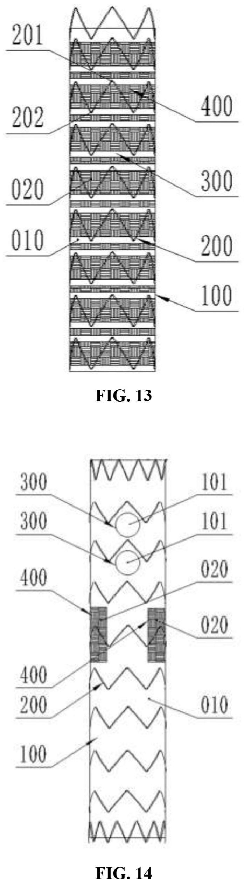

Referring to FIG. 12 and FIG. 13, multiple the circumferential struts are arranged at intervals along an axial direction of the stent 200, and the in-situ fenestration region 400 is arranged between the two adjacent circumferential struts. Therefore, the region between two adjacent circumferential struts can also be utilized for the in-situ fenestration, and there will be no exposure of the circumferential struts at the fenestration site, thereby avoiding the stimulation of the vascular wall by the exposed circumferential struts.

Optionally, multiple fenestration-friendly areas 020 are arranged at intervals along the axial direction of the stent 200, and multiple fenestration-friendly areas 020 respectively extend along a circumference of the stent 200. The fenestration-friendly area 020 is in the shape of a ring or a semi-circular ring. The axial dimensions of a part of the fenestration-friendly areas 020 are smaller than the spacing between adjacent circumferential struts, thereby utilizing the area between the two circumferential struts; and the axial dimensions of the other part of the fenestration-friendly areas 020 are smaller than the axial spacing between the peak 201 and the valley 202 of the support rib on the stent 200, so that the fenestration-friendly area 020 is located at the middle part of the support rib. Therefore, the peaks 201 and the valleys 202 can still be joined with the basic area 010. By providing this structure in the stent graft, it ensures the blood supply of the intercostal artery and other branch vessels when expanding the true lumen, thereby preventing complications such as paraplegia. It ensures the blood supply to the branch vessels and enables less stimulation to the vessel walls. The original graft structure will not be damaged, and the mechanical properties and fatigue resistance can be better. The wave peaks 201 and wave valleys 202 are joined to the basic area 010, which also helps to reduce the stimulation to the vessel walls, and improves the mechanical properties such as fatigue resistance of the stent graft.

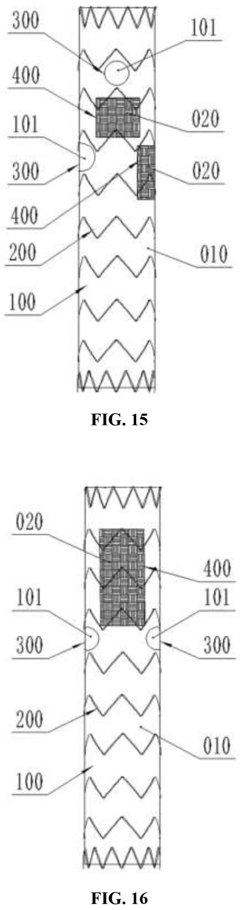

As shown in FIG. 14, FIG. 15, FIG. 16, and FIG. 17, the stent graft has four branch regions in total, including a left renal artery branch region, a right renal artery branch region, an inferior mesenteric artery branch region, and a visceral artery branch region, where at least one of the four branch regions is configured as one of the fenestration-friendly areas 020, and the remaining branch regions are configured as pre-fenestration regions 300. The stent graft can, according to individual anatomical characteristics of patient, have yarn weaving structures covering two of fenestration-friendly area 020 in the left renal artery branch region, the right renal artery branch region, the inferior mesenteric artery branch region, and the visceral artery branch region, thereby facilitating the in-situ fenestration operation at these locations. The arrangement of the in-situ fenestration region can accommodate the variable lesion vascular structures of the patients, thereby improving the applicability and selectivity of the stent. Referring to FIG. 15, the pre-fenestration adopted for positioning in the bilateral renal artery branch region, and then the inferior mesenteric artery branch region and the visceral artery branch region are configured as the fenestration-friendly areas 020 to facilitate the in-situ fenestration in the inferior mesenteric artery branch region and the visceral artery branch region.

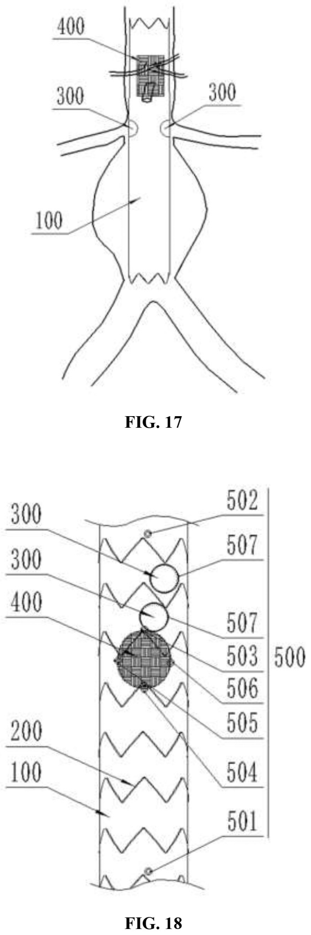

Referring to FIG. 18, based on any one of the above embodiments, the stent graft is provided with a radiopaque device 500. Through the radiopaque device 500, the implantation location of the stent graft can be confirmed though X-ray. The radiopaque device 500 can be implemented in the following various embodiments. At least one form thereof can be optionally disposed on the stent graft, and it optionally includes a combination of multiple forms.

In some embodiments, the radiopaque device can include: a first radiopaque part 501 and a second radiopaque part 502. One of the first radiopaque part 501 and the second radiopaque part 502 is connected to the proximal end of the stent graft, and the other is connected to the distal end of the stent graft. By detecting the locations of the first radiopaque part 501 and the second radiopaque part 502, the implantation location of the stent graft can be determined.

In some embodiments, the radiopaque device can include: a third radiopaque part 503 and a fourth radiopaque part 504. Each in-situ fenestration region 400 is connected to the third radiopaque part 503 at the distal end, and each in-situ fenestration region 400 is connected to the fourth radiopaque part 504 at the proximal end. By detecting the positions of the third radiopaque part 503 and the fourth radiopaque part 504, the implantation location of the in-situ fenestration region 400 in the axial direction of the stent graft can be determined.

In some embodiments, the radiopaque device can include: a fifth radiopaque part 505 and a sixth radiopaque part 506. The in-situ fenestration region 400 along the circumference of the stent grafts is located between the fifth radiopaque part 505 and the sixth radiopaque part 506. By detecting the positions of the fifth radiopaque part 505 and the sixth radiopaque part 506, the implantation location of the in-situ fenestration region 400 in the circumference of the stent graft can be determined.

In some embodiments, the radiopaque device can include seventh radiopaque parts 507. Multiple seventh radiopaque parts 507 are arranged at intervals around the pre-fenestration region 300, or the seventh radiopaque parts 507 extend continuously around the pre-fenestration region 300. The implantation location of the pre-fenestration region 300 can be determined by detecting the seventh radiopaque parts 507.

In the above embodiment, the first radiopaque part 501, the second radiopaque part 502, the third radiopaque part 503, the fourth radiopaque part 504, the fifth radiopaque part 505, the sixth radiopaque part 506, and the seventh radiopaque part 507 can be configured as structures such as dots or rings. The radiopaque parts can be made from materials including gold or platinum-iridium alloy, and can be connected to the graft 100 by suturing.

As shown in FIG. 1, FIG. 2, FIG. 3, and FIG. 4, the graft manufacturing method provided by the embodiments of the present disclosure can be used to manufacture the graft 100 separately, or can be used to weave the graft 100 on the stent 200. The steps includes: forming a graft 100 by interweaving multiple warp yarns 110 and multiple weft yarns 120, and providing the graft 100 with a basic area 010 and at least one fenestration-friendly area 020; in the basic area 010, making any one of warp yarns 110 reverse direction once between the abluminal layer and the luminal layer after being interwoven with j weft yarns 120, and making any one of weft yarns 120 reverse direction once between the abluminal layer and the luminal layer after being interwoven with k warp yarns 110; and in the fenestration-friendly area 020, selecting at least one of the warp yarns 110 to reverse direction once between the abluminal layer and the luminal layer after being interwoven with m weft yarns 120, and selecting at least one of the weft yarns 120 to reverse direction once between the abluminal layer and the luminal layer after being interwoven with n warp yarns 110, wherein one of m and n is greater than one of j and k, and is greater than or equal to the other of j and k; and the other of m and n is greater than or equal to j, and is greater than or equal to k. With reference to prior textile processes, the interweaving pattern of the warp yarns 110 and weft yarns 120 is limited by the process described above. Any textile process that satisfies the above interweaving procedure can be used. The textile process will not be elaborated further herein. The graft 100 formed by this process has the technical effects described in the above embodiments. In addition, yarns with lower bending stiffness can be used in the fenestration-friendly area 020, thereby making the yarns of this region more susceptible to deformation and the fenestration process easier. An elastic film (such as TPU film, EPTFE film, or silicone) can further be coated on the fenestration-friendly area 020, thereby reducing the risk of endoleak.

In the graft manufacturing method, the positions of multiple branch regions can be first determined based on an implantation location of the stent graft; then the fenestration-friendly area 020 at a position corresponding to at least one of the branch regions is formed; the remaining regions are configured as the basic area 010; and an opening 101 at a position corresponding to remaining branch regions is reserved. Therefore, the graft 100 and the stent graft accommodating the physiological structure of the branch vessels of different patients can be customized.

Finally, it should be noted that the above embodiments are only used to illustrate the technical solutions of the present disclosure, and not to restrict it. Although the present disclosure is described in detail with reference to each foregoing embodiments, it should be understood by persons of ordinary skill in the art that they can still modify the technical solutions described in the foregoing embodiments or make equivalent substitutions for some or all of the technical features, and these modifications or substitutions do not take the essence of the corresponding technical solutions out of the scope of the technical solutions in each embodiment of the present disclosure.

### Industrial Applicability

A graft with a basic area and at least one fenestration-friendly area is provided. The graft is formed by multiple warp yarns and multiple weft yarns, and multiple warp yarns and multiple weft yarns are interwoven. The multiple warp yarns and the multiple weft yarns extend reciprocally to the abluminal layer and the luminal layer. In the basic area, any one of the warp yarns reverses direction once between the abluminal layer and the luminal layer when interwoven with j weft yarns, and any one of the weft yarns reverses direction once between the abluminal layer and the luminal layer when interwoven with k warp yarns; and in the fenestration-friendly area, at least one of the warp yarns reverses direction once between the abluminal layer and the luminal layer when interwoven with m weft yarns, and at least one of the weft yarns reverses direction once between the abluminal layer and the luminal layer when interwoven with n warp yarns, wherein one of m and n is greater than one of j and k, and is greater than or equal to the other of j and k; and the other of m and n is greater than or equal to j, and is greater than or equal to k. As a result, the fenestration-friendly area facilitates the graft puncture and fenestration. The success rate of in-situ fenestration is higher, fragmentation and detachment of the graft caused by graft puncture can be avoided, and distal embolism can be prevented. The yarn structures around the opening of the in-situ fenestration tends to tighten, which is beneficial for reinforcing the graft tissue around the opening and reducing the occurrence of endoleak. Moreover, it is easy to achieve diversified designs for the position, number, and shape of the opening of the in-situ fenestration, which can adapt to the variable physiological structures of the branch vessels of patients.

## Claims

1. A graft, having an abluminal layer and a luminal layer, and configured to cover a stent (200), wherein the graft (100) is arranged with a basic area (010) and at least one fenestration-friendly area (020);
the graft (100) is formed by multiple warp yarns (110) and multiple weft yarns (120), the multiple warp yarns (110) and the multiple weft yarns (120) are interwoven, and the multiple warp yarns (110) and the multiple weft yarns (120) extend reciprocally to the abluminal layer and the luminal layer;
in the basic area (010), any one of the warp yarns (110) reverses direction once between the abluminal layer and the luminal layer when interwoven with j weft yarns (120), and any one of the weft yarns (120) reverse direction once between the abluminal layer and the luminal layer when interwoven with k warp yarns (110); and
in the fenestration-friendly area (020), at least one of the warp yarns (110) reverses direction once between the abluminal layer and the luminal layer when interwoven with m weft yarns (120), and at least one of the weft yarns (120) reverses direction once between the abluminal layer and the luminal layer when interwoven with n warp yarns (110), wherein
one of m and n is greater than one of j and k, and is greater than or equal to the other of j and k; and the other of m and n is greater than or equal to j, and is greater than or equal to k.

2. The graft according to claim 1, wherein the basic area (010) is configured as a plain weave structure; and
in the fenestration-friendly area (020), at least one of m and n is greater than 1.

3. The graft according to claim 1 or 2, wherein, on any one of projection planes perpendicular to the warp yarns (110) within the fenestration-friendly area (020), the number of the warp yarns (110) from the basic area (010) is equal to the number of the warp yarns (110) from the fenestration-friendly area (020), and the warp yarns (110) of the basic area (010) extend continuously with the warp yarns (110) of the fenestration-friendly area (020); and
on any one of projection planes perpendicular to the weft yarns (120) within the fenestration-friendly area (020), the number of the weft yarns (120) of the basic area (010) is equal to the number of the weft yarns (120) of the fenestration-friendly area (020), and the weft yarns (120) of the basic area (010) extend continuously with the weft yarns (120) of the fenestration-friendly area (020).

4. The graft according to any one of claims 1-3, wherein bending stiffness of the warp yarns (110) in the fenestration-friendly area (020) is less than bending stiffness of the warp yarns (110) in the basic area (010), and/or, the bending stiffness of the weft yarns (120) in the fenestration-friendly area (020) is less than the bending stiffness of the weft yarns (120) in the basic area (010).

5. The graft according to any one of claims 1-4, wherein yarn fineness of the warp yarns (110) in the fenestration-friendly area (020) is less than yarn fineness of the warp yarns (110) in the basic area (010).

6. The graft according to any one of claims 1-5, wherein a number of multifilament filaments of the warp yarns (110) in the fenestration-friendly area (020) is less than a number of multifilament filaments of the warp yarns (110) in the basic area (010).

7. The graft according to any one of claims 1-6, wherein a density of the warp yarns (110) in the fenestration-friendly area (020) is less than a density of the warp yarns (110) in the basic area (010).

8. The graft according to any one of claims 1-7, wherein elastic films are coated on woven fabric surfaces formed by interweaving the warp yarns (110) and the weft yarns (120) in the fenestration-friendly area (020).

9. A stent graft, comprising: a stent (200) and the graft according to any one of claims 1-8, wherein the graft (100) covers the stent (200);
the stent graft has at least one in-situ fenestration region (400); and
the fenestration-friendly area (020) covers the in-situ fenestration region (400).

10. The stent graft according to claim 9, wherein the stent graft has at least one pre-fenestration region (300); and an opening (101) of the graft (100) is formed at a position of the pre-fenestration region (300).

11. The stent graft according to claim 9 or 10, wherein the stent (200) is joined to the basic area (010); or
an axial length of an attachment of the stent (200) to the basic area (010) is greater than an axial length of the attachment of the stent (200) to the fenestration-friendly area (020).

12. The stent graft according to any one of claims 9-11, wherein the stent (200) comprises circumferential struts extending along a circumference of the stent (200); the circumferential struts are bent to form peaks (201) and valleys (202); and
the basic area (010) covers and joins the peaks (201) and the valleys (202).

13. The stent graft according to claim 12, wherein multiple circumferential struts are arranged at intervals along an axial direction of the stent (200), and
the in-situ fenestration region (400) is arranged between the two adjacent circumferential struts.

14. The stent graft according to any one of claims 9-13, wherein multiple fenestration-friendly areas (020) are arranged at intervals along the axial direction of the stent (200), and the multiple fenestration-friendly areas (020) respectively extend along the circumference of the stent (200).

15. The stent graft according to any one of claims 9-14, wherein the stent graft has three branch regions, comprising: a brachiocephalic artery branch region, a common carotid artery branch region, and a subclavian artery branch region, wherein
at least one of the brachiocephalic artery branch region, the common carotid artery branch region, and the subclavian artery branch region is configured as a pre-fenestration region (300); and the fenestration-friendly areas (020) cover the remaining branch regions; or
the fenestration-friendly area (020) covers the brachiocephalic artery branch region, the common carotid artery branch region, and the subclavian artery branch region.

16. The stent graft according to any one of claims 9-15, wherein the stent graft has four branch regions, comprising a left renal artery branch region, a right renal artery branch region, an inferior mesenteric artery branch region, and a visceral artery branch region, wherein
at least one of the four branch regions is configured as one of the fenestration-friendly areas (020), and the remaining branch regions are configured as the pre-fenestration regions (300).

17. The stent graft according to any one of claims 9-16, wherein at least one of the fenestration-friendly areas (020) extends along the circumference or axial direction of the stent (200) and covers at least one of the in-situ fenestration regions (400).

18. The stent graft according to any one of claims 9-17, wherein the stent graft is provided with a radiopaque device (500), wherein
the radiopaque device (500) comprises a first radiopaque part (501) and a second radiopaque part (502), wherein one of the first radiopaque part (501) and the second radiopaque part (502) is connected to a proximal end of the stent graft, and the other is connected to a distal end of the stent graft; or
the radiopaque device (500) comprises a third radiopaque part (503) and a fourth radiopaque part (504), wherein each of the distal end of the in-situ fenestration regions (400) is connected to the third radiopaque part (503), and each of the proximal end of the in-situ fenestration regions (400) is connected to a fourth radiopaque part (504); or
the radiopaque device (500) comprises a fifth radiopaque part (505) and a sixth radiopaque part (506), wherein the in-situ fenestration regions (400) along the circumference of the stent graft are located between the fifth radiopaque part (505) and the sixth radiopaque part (506); or
the radiopaque device (500) comprises multiple seventh radiopaque parts (507), and the multiple seventh radiopaque parts 507 are arranged at intervals around the pre-fenestration region (300).

19. A graft manufacturing method, comprising following steps:
forming a graft (100) by interweaving multiple warp yarns (110) and multiple weft yarns (120), and providing the graft (100) with a basic area (010) and at least one fenestration-friendly area (020);
making, in the basic area (010), any one of the warp yarns (110) reverse direction once between an abluminal layer and a luminal layer after being interwoven with j weft yarns (120), and making any one of the weft yarns (120) reverse direction once between the abluminal layer and the luminal layer after being interwoven with k warp yarns (110); and
selecting, in the fenestration-friendly area (020), at least one of the warp yarns (110) to reverse direction once between the abluminal layer and the luminal layer after being interwoven with m weft yarns (120), and selecting at least one of the weft yarns (120) to reverse direction once between the abluminal layer and the luminal layer after being interwoven with n warp yarns (110), wherein
one of m and n is greater than one of j and k, and is greater than or equal to the other of j and k; and the other of m and n is greater than or equal to j, and is greater than or equal to k.

20. The graft manufacturing method according to claim 19, further comprising following steps:
determining positions of multiple branch regions based on an implantation location of the stent graft;
forming the fenestration-friendly area (020) at a position corresponding to at least one of the branch regions; configuring remaining regions as the basic area (010); and reserving an opening (101) at a position corresponding to remaining branch regions.
